# EUROPÄISCHE PATENTANMELDUNG

(11) **EP 0 545 866 A1**
(43) Veröffentlichungstag der Anmeldung: **09.06.1993**
(21) Anmeldenummer: 92810923.0
(22) Anmeldetag: 27.11.1992
(51) Int. Cl.: C07C 201/12, C07C 205/37, C07C 205/59, C07C 201/08

(54) **Verfahren zur Herstellung von Trifluorbenzolderivaten**

(30) Priorität: 06.12.1991 CH 3594/91
(71) Anmelder: CIBA-GEIGY AG, CH-4002 Basel (CH)
(72) Erfinder: Szczepanski, Henry, Dr., CH-4323 Wallbach (CH); Burckhardt, Urs, Dr., CH-4051 Basel (CH); Pascual, Alfons, Dr., CH-4053 Basel (CH); Farooq, Saleem, Dr., CH-4422 Arisdorf (CH)

(57) **Zusammenfassung**

Ein Verfahren zur Herstellung von Trifluornitrobenzolderivaten der Formel 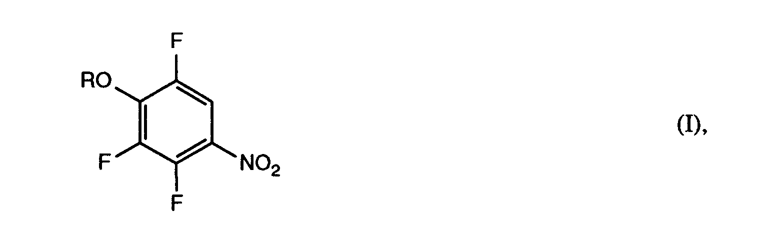
worin R Wasserstoff, C₁-C₈-Alkyl, Halogen-C₁-C₈-alkyl oder Allyl bedeutet, sowie ein Verfahren zur Herstellung von 2-Nitro-3,4,6-trifluorbenzoesäurederivaten der Formel 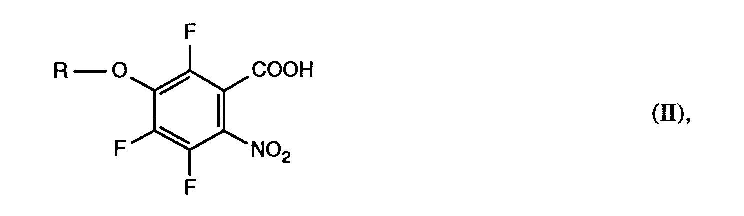
worin R die oben gegebenen Bedeutungen hat, werden beschrieben.

Verbindungen der Formel I sind wertvolle Zwischenprodukte zur Herstellung von Wirkstoffen für die Schädlingsbekämpfung.

## Beschreibung

Die vorliegende Erfindung betrifft ein neuartiges Verfahren zur Herstellung von 2,3,5-Trifluornitrobenzolderivaten sowie neue Zwischenprodukte.

Gegenstand der Erfindung ist ein Verfahren zur Herstellung von 2,3,5-Trifluornitrobenzolderivaten der Formel 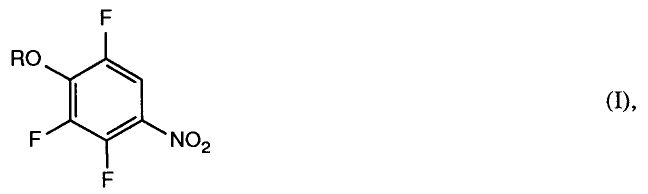
worin R Wasserstoff, C₁-C₈-Alkyl, Halogen-C₁-C₈-alkyl oder Allyl bedeutet.

Die Verbindungen der Formel I sind wertvolle Zwischenprodukte zur Herstellung von hochaktiven insektizid und akarizid wirksamen Substanzen aus der Klasse der Benzoylharnstoffe. Diese Endprodukte sind aus der EP-A-327 504 bekannt. Das in der EP-A-327 504 offenbarte Verfahren ist jedoch für die grosstechnische Herstellung des benötigten Zwischenprodukts wegen zu geringer Ausbeute und anfallenden Nebenprodukten wenig geeignet. Es besteht daher Bedarf nach einem besser geeigneten Syntheseweg zur Herstellung der Verbindungen der Formel I. Das erfindungsgemäss vorgeschlagene neue Verfahren erfüllt diese Anforderungen überraschenderweise weitgehend.

In der obigen Definition der Formel I ist unter Alkyl beispielsweise Methyl, Ethyl, Propyl, Isopropyl, Butyl und seine Isomeren, Amyl, Isoamyl oder n-Octyl zu verstehen.

Die als Substituenten in Betracht kommenden ein- oder mehrfach durch Halogen substituierten C₁-C₈-Alkyle können teilweise oder auch perhalogeniert sein, wobei für die Alkyle die oben gegebenen Definitionen gelten. Unter Halogen wird Fluor, Chlor, Brom oder Jod, insbesondere Fluor oder Chlor verstanden. Geeignete Beispiele solcher Substituenten sind u.a. CF₃, das ein- bis fünffach durch Fluor, Chlor und/oder Brom substituierte Ethyl wie z.B. CHFCF₃, CF₂CF₃, CF₂CCl₃, CF₂CHCl₂, CF₂CHF₂, CF₂CFCl₂, CF₂CHBr₂, CF₂CHClF, CH₂CF₃, CF₂CH₂Cl, CF₂CHBr₂, CFClCFCl, CF₂CHBrF oder CCIFCHCIF; das ein- bis siebenfach durch Fluor, Chlor und/oder Brom substituierte Propyl oder Isopropyl wie z.B. CF₂CHBrCH₂Br, CF₂CHFCF₃, CHFCF₂CF₃, CF₂CF₂CF₃, CH₂CF₂CF₃ oder CHFCF₂CF₃; das ein- bis neunfach durch Fluor, Chlor und/oder Brom substituierte Butyl oder eines seiner Isomeren wie z.B. CHFCF₂CHFCF₃, (CF₂)₃CF₃ oder CH₂CF₂CF₂CF₃.

Die nach dem erfindungsgemässen Verfahren hergestellten Trifluornitrobenzolderivate der Formel I sind teilweise bekannt. Es handelt sich dabei um wertvolle Zwischenprodukte für die Herstellung von Aktivstoffen welche eine ausgeprägte insektizide und akarizide Wirkung haben. Als solche Wirkstoffe seien beispielsweise genannt:
N-2,6-Difluorbenzoyl-N'-(4-ethoxy-2,3,5-trifluor-phenyl)-harnstoff,
N-2,6-Difluorbenzoyl-N'-(4-isopropoxy-2,3,5-trifluor-phenyl)-harnstoff,
N-2,6-Difluorbenzoyl-N'-[4-(2-chlor-1,1,2-trifluorethoxy)-2,3,5-trifluor-phenyl]-harnstoff,
N-2,6-Difluorbenzoyl-N'-(4-tert.-butoxy-2,3,5-trifluor-phenyl)-harnstoff,
N-2,6-Difluorbenzoyl-N'-[4-(1,1,2,2-tetrafluorethoxy)-2,3,5-trifluor-phenyl]-harnstoff,
N-2,6-Difluorbenzoyl-N'-[4-(1,1,2,3,3,3-hexafluorpropoxy)-2,3,5-trifluor]-phenylharnstoff,
N-2,6-Difluorbenzoyl-N'-(4-trifluormethoxy-2,3,5-trifluor-phenyl)-harnstoff,
N-2,6-Difluorbenzoyl-N'-(4-heptafluorpropoxy-2,3,5-trifluor-phenyl)-harnstoff,
N-2,6-Difluorbenzoyl-N'-(4-nonafluorbutoxy-2,3,5-trifluor-phenyl)-harnstoff,
N-2,6-Difluorbenzoyl-N'-[4-(2,2,2-trifluorethoxy)-2,3,5-trifluor-phenyl]-harnstoff,
N-2,6-Difluorbenzoyl-N'-[4-(2,2-dibrom-1,1-difluorethoxy)-2,3,5-trifluor-phenyl]-harnstoff,
N-2,6-Difluorbenzoyl-N'-[4-(1,2-dichlor-1,2-difluorethoxy)-2,3,5-trifluor-phenyl]-harnstoff,
N-2,6-Difluorbenzoyl-N'-[4-(2-chlor-1,1-difluorethoxy)-2,3,5-trifluor-phenyl]-harnstoff,
N-2,6-Difluorbenzoyl-N'-[4-(2,2,3,3,3-pentafluorpropoxy)-2,3,5-trifluor-phenyl]-harnstoff,
N-2,6-Difluorbenzoyl-N'-[4-(heptafluorpropoxy)-2,3,5-trifluor-phenyl]-harnstoff,
N-2,6-Difluorbenzoyl-N'-[4-(2,2,3,3,4,4,4-heptafluorbutoxy)-2,3,5-trifluor-phenyl]-harnstoff,
N-2,6-Difluorbenzoyl-N'-[4-(nonafluorbutoxy)-2,3,5-trifluor-phenyl]-harnstoff, und
N-2-Chlor-6-fluorbenzoyl-N'-[4-(1,1,2,3,3,3-hexafluorpropoxy)-2,3,5-trifluor-phenyl]-harnstoff.

Erfindungsgemäss wird vorgeschlagen, die 2,3,5-Trifluornitrobenzolderivate der Formel 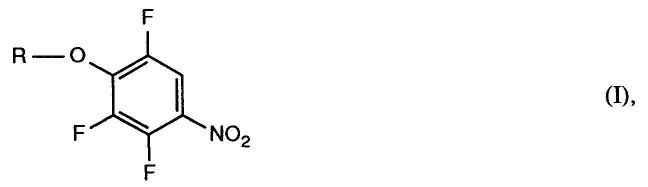
worin
R Wasserstoff, C₁-C₈-Alkyl, Halogen-C₁-C₈-alkyl oder Allyl bedeutet, herzustellen, indem man eine 3,4,6-Trifluor-2-nitrobenzoesäure der Formel II 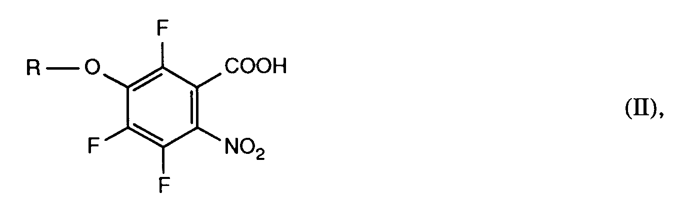
worin
R Wasserstoff, C₁-C₈-Alkyl, Halogen-C₁-C₈-alkyl oder Allyl bedeutet, oder eines ihrer Salze, decarboxyliert.

Mit Vorteil führt man die Decarboxylierung entweder
a) in Gegenwart einer Base; oder
b) in einem organischen Lösungsmittel, einem organischen Lösungsmittel/Wasser-Gemisch oder in Wasser; oder
c) bei +50°C bis +250°C durch.

Besonders bevorzugt ist eine Ausführungsform, bei der man in einem organischen Lösungsmittel, einem organischen Lösungsmittel/Wassergemisch oder in Wasser; in Gegenwart einer Base und bei +50°C bis +250°C arbeitet.

Als organische Lösungsmittel für die Decarboxylierungsreaktion (II→I) des erfindungsgemässen Verfahrens eignen sich dabei insbesondere Sulfoxide wie Dimethylsulfoxid oder Sulfolan; Amide wie Formamid, Dimethylformamid, N-Methylacetamid oder Dimethylacetamid; N-Methylpyrrolidon; Alkohole wie n-Butanol, iso-Butanol, n-Amylalkohol, n-Octanol, Ethylenglykol, Ethylenglykolmonomethylether, Ethylenglykolmonoethylether oder Diethylenglykoldimethylether, Tetramethylharnstoff; aromatische Kohlenwasserstoffe wie Toluol oder Xylol; halogenierte Aromaten wie Chlorbenzol oder 1,2-Dichlorbenzol; Nitrobenzol; Tetrahydronaphthalin; Decalin; heterozyklische Lösungsmittel wie Pyridin oder Chinolin.

Als Basen kommen sowohl anorganische als auch organische Basen in Frage. Unter den anorganischen Basen seien hervorgehoben Karbonate wie Natriumkarbonat, Natriumhydrogenkarbonat oder Kaliumkarbonat; Phosphate wie Natriumphosphat oder Natriumhydrogenphosphat; Natriumhydroxid, Kaliumhydroxid, Calciumhydroxid oder Magnesiumhydroxid.

Unter den organischen Basen seien sekundäre und tertiäre Amine, insbesondere Triethylamin, Triisopropanolamin, Triethanolamin, Tripropylamin, Tributylamin, N,N-Dimethylbenzylamin, Diisopropylamin und N,N-Dimethylanilin; Alkoholate wie Natriummethylat, Na-ethylat oder Kalium-tert.-Butylat; sowie Pyridin genannt.

Ganz besonderes Interesse beansprucht jedoch ein Verfahren, bei dem die Decarboxylierung in Dimethylsulfoxid, in Gegenwart von Triethylamin bei +80°C bis +140°C durchgeführt wird.

Die erfindungsgemässe Decarboxylierung kann sowohl mit dem freien Phenol der Formel 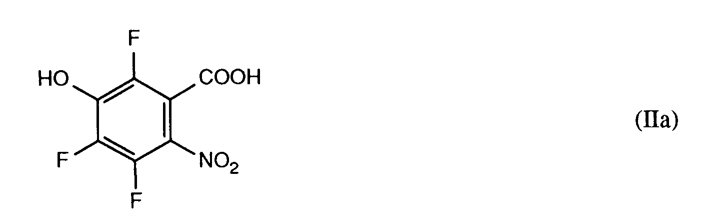
als auch mit der veretherten Verbindung 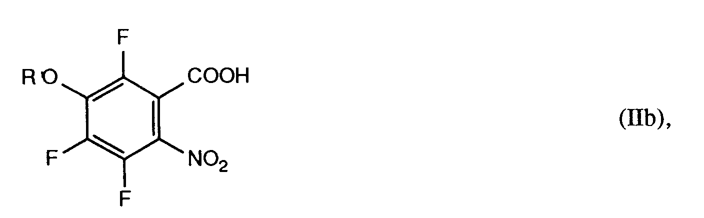
worin R'C₁-C₈-Alkyl, Halogen-C₁-C₈-alkyl oder Allyl bedeutet, durchgeführt werden.

Bei der Decarboxylierung der Substanz der Formel IIa erhält man ein Phenol der Unterformel Ia 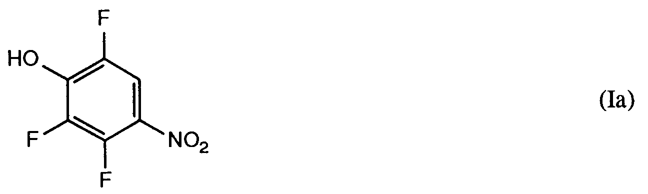
welches gewünschtenfalls durch Veretherung in eine Verbindung der Formel 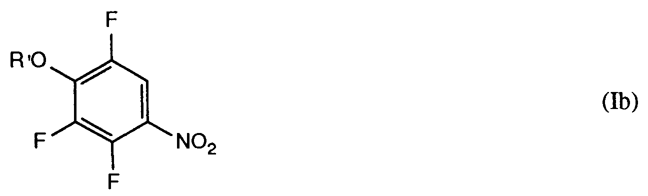
übergeführt werden kann, wobei die Bedeutung von R' in Ib die gleiche ist wie in IIb.

Andererseits kann man das freie Nitrophenol Ia herstellen, indem man eine veretherte Carbonsäure der Formel IIb decarboxyliert und die so erhaltene Zwischenstufe Ib durch Etherspaltung in das freie Phenol der Formel Ia überführt.

Die Veretherungsreaktionen (Ia→Ib) werden nach literaturbekannten Methoden durchgeführt, wobei als Alkylierungsmittel beispielsweise Alkylhalogenide wie Methylchlorid, -bromid, -jodid, Ethylchlorid, -bromid oder -jodid oder tert.-Butylchlorid; Jodfluoralkane wie CF₃CF₂J oder CF₃CF₂CF₂J; Alkene wie Ethylen, Propen, 2-Buten oder Isopropen;
Alkylsulfate wie Dimethylsulfat oder Diethylsulfat; halogenierte Alkene wie Tetrafluorethen, Hexafluorpropen oder Chlortrifluorethen; sowie Chlorfluoralkyle wie CF₂Cl₂, CF₃Cl, ClFCH-CF₂-CF₃ oder ClFCH-(CF₂)₅-CF₃; und Allylchlorid, -bromid oder -jodid in Frage kommen.

Auch die Etherspaltung (Ib→Ia) wird nach an sich bekannten Methoden durchgeführt, beispielsweise in Gegenwart starker Mineralsäuren wie Jodwasserstoff- und Bromwasserstoffsäure.

Die in der Literatur noch nicht beschriebenen Verbindungen der Formel I, worin R Halogen-C₁-C₈-alkyl oder Allyl bedeutet, bilden ebenfalls einen Gegenstand der Erfindung. Es handelt sich insbesondere um die Verbindungen
4-(1,1,2,3,3,3-Hexafluorpropoxy)-2,3,5-trifluornitrobenzol,
4-(1,1,2,2-Tetrafluorethoxy)-2,3,5-trifluornitrobenzol,
4-(2-Chlor-1,1,2-trifluorethoxy)-2,3,5-trifluornitrobenzol,
4-(1,2,3,3,3,-Pentafluorpropoxy)-2,3,5-trifluornitrobenzol,
4-Heptafluorpropoxy-2,3,5-trifluornitrobenzol,
4-(2,2,2-Trifluorethoxy)-2,3,5-trifluornitrobenzol,
4-(2,2-Dibrom-1,1-difluorethoxy)-2,3,5-trifluornitrobenzol,
4-(1,2-Dichlor-1,2-difluorethoxy)-2,3,5-trifluornitrobenzol,
4-(2-Chlor-1,1-difluorethoxy)-2,3,5-trifluornitrobenzol,
4-(2,2,3,3,3-Pentafluorpropoxy)-2,3,5-trifluornitrobenzol,
4-(2,2,3,3,4,4,4-Heptafluorbutoxy)-2,3,5-trifluornitrobenzol,
4-Nonafluorbutoxy-2,3,5-trifluornitrobenzol,
4-Trifluormethoxy-2,3,5-trifluornitrobenzol und
4-Chlordifluormethoxy-2,3,5-trifluornitrobenzol.

Ein weiterer Gegenstand der vorliegenden Erfindung beinhaltet ein Verfahren zur Herstellung von 2-Nitro-3,4,6-trifluorbenzoesäurederivaten der Formel (II), dadurch gekennzeichnet, dass man ein 2,4,5-Trifluorbenzoesäurederivat der Formel 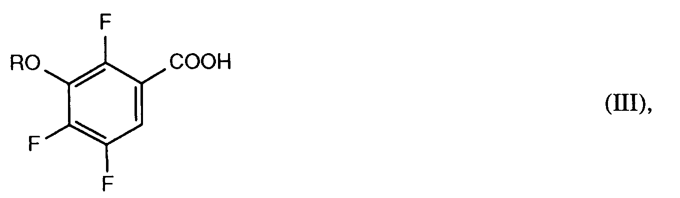
worin
R Wasserstoff, C₁-C₈-Alkyl, Halogen-C₂-C₈-alkyl oder Allyl bedeutet, mit einem Nitrierungsmittel umgesetzt.

Die Nitrierung (III→II) wird nach aus der Literatur an sich bekannten Methoden durchgeführt, vorzugsweise in Gegenwart eines organischen Lösungsmittels, gegebenenfalls in Gegenwart einer Säure.

Als Nitrierungsmittel eignen sich die üblicherweise für solche Reaktionen eingesetzten Reagenzien, wie Salpetersäure, N₂O₃, N₂O₄, N₂O₅, Alkalimetall-Nitrate wie KNO₃ oder NaNO₃, AgNO₃, Alkylnitrate wie Ethyl- oder Butylnitrat und Nitroniumsalze wie NO₂BF₄ oder NO₂CF₃SO₃.

Als Lösungsmittel kommen insbesondere halogenierte Kohlenwasserstoffe, wie Chloroform, Methylenchlorid, 1,2-Dichlorethan, 1,1,1-Trichlorethan oder 1,1,2-Trichlorethan; Nitromethan; Nitrobenzol; halogenierte Aromaten, wie Chlorbenzol oder ortho-Dichlorbenzol; Ether, wie Diethylether, Di-n-butylether, tert.-Butylmethylether, Tetrahydrofuran, Dioxan oder Ethylenglykoldimethylether, und Wasser sowie Mischungen verschiedener Lösungsmittel in Frage. Besonders bevorzugte Lösungsmittel sind einerseits halogenierte Kohlenwasserstoffe, insbesondere Chloroform, und andererseits Wasser.

Vorzugsweise werden bei den erfindungsgemässen Nitrierungen die üblichen Säuren zugesetzt, also beispielsweise Schwefelsäure, Perchlorsäure, Methansulfonsäure, Trifluormethansulfonsäure oder Phosphorsäure; ebenfalls Lewis-Säuren wie FeCl₃, BF₃, AlCl₃ oder SnCl₄. Besonders bevorzugt ist jedoch ein Verfahren, bei dem man mit Nitriersäure, ganz besonders nur mit Salpetersäure nitriert.

Andererseits werden Verfahrensweisen bevorzugt, bei denen man
a) bei 0-60°C; oder
b) mit Salpetersäure, in Gegenwart eines Lösungsmittels und bei 0°C bis +60°C; oder
c) mit Salpetersäure, in Chloroform und bei +10°C bis +50°C nitriert.

Bei der Synthese der Produkte der Formel II können folgende Verfahrensvarianten beschritten werden:

### A) 3-Hydroxy-2,4,5-trifluorbenzoesäure der Formel

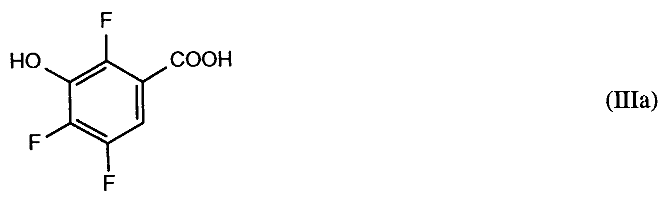

kann durch Nitrierung in 5-Hydroxy-2-nitro-3,4,6-trifluorbenzoesäure der Formel IIa übergeführt und dann gegebenenfalls verethert werden, um zum Produkt der Formel IIb zu gelangen, oder

### B) ein Produkt der Formel

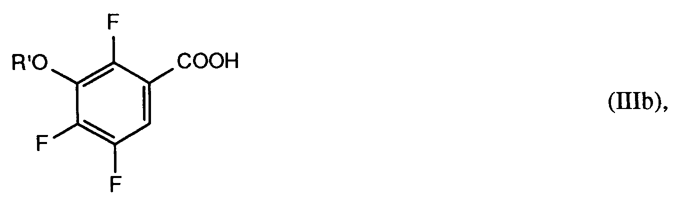

worin
R'C₁-C₈-Alkyl, Halogen-C₁-C₈-alkyl oder Allyl bedeutet, kann durch Nitrierung in eine Verbindung der Formel IIb, und dieses Produkt gewünschtenfalls durch Etherspaltung in 5-Hydroxy-2-nitro-3,4-6-trifluor-benzoesäure der Formel IIa überführt werden.

Die Veretherungsreaktionen (IIa-IIb) werden nach literaturbekannten Methoden durchgeführt, wobei als Alkylierungsmittel beispielsweise Alkylhalogenide wie Methylchlorid, -bromid, -jodid, Ethylchlorid, -bromid oder -jodid oder tert.-Butyl-chlorid; Jodfluoralkyle wie CF₃CF₂J oder CF₃CF₂CF₂-J; Alkene wie Ethylen, Propen, 2-Buten oder Isopropen; Alkylsulfate wie Dimethylsulfat oder Diethylsulfat; halogenierte Alkene wie Tetrafluorethen, Hexafluorpropen oder Chlortrifluorethen; sowie Chlor-Fluoralkyle CF₂Cl₂, CF₃Cl, ClFCH-CF₂-CF₃ oder ClFCH-(CF₂)₅-CF₃; und Allylchlorid, -bromid oder -jodid in Frage kommen.

Auch die Etherspaltung (IIb→IIa) wird nach an sich bekannten Methoden durchgeführt, beispielsweise in Gegenwart starker Mineralsäuren wie Jodwasserstoff- und Bromwasserstoffsäure.

Die Verbindungen der Formel 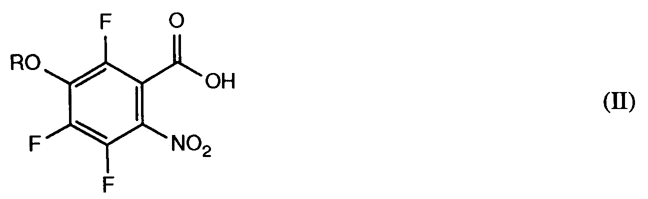
worin R Wasserstoff, C₁-C₈-Alkyl, Halogen-C₁-C₈-alkyl oder Allyl bedeutet, sowie deren Salze, sind neu und bilden ebenfalls einen Gegenstand der vorliegenden Erfindung. Bevorzugte Einzelverbindungen aus dieser Gruppe sind:
5-Methoxy-2-nitro-3,4,6-trifluorbenzoesäure,
5-Ethoxy-2-nitro-3,4,6-trifluorbenzoesäure,
5-Propoxy-2-nitro-3,4,6-trifluorbenzoesäure,
5-Isopropoxy-2-nitro-3,4,6-trifluorbenzoesäure,
5-Butoxy-2-nitro-3,4,6-trifluorbenzoesäure,
5-tert.-Butoxy-2-nitro-3,4,6-trifluorbenzoesäure,
5-(1,1,1,2,3,3,3-Hexafluorpropoxy)-2-nitro-3,4,6-trifluorbenzoesäure,
5-(1,1,2,2-Tetrafluorethoxy)-2-nitro-3,4,6-trifluorbenzoesäure,
5-(2-Chlor-1,1,2-trifluorethoxy)-2-nitro-3,4,6-trifluorbenzoesäure,
5-Heptafluorpropoxy-2-nitro-3,4,6-trifluorbenzoesäure,
5-(1,2,3,3,3-Pentafluorpropoxy)-2-nitro-3,4,6-trifluorbenzoesäure,
5-(2,2,2-Trifluorethoxy)-2-nitro-3,4,6-trifluorbenzoesäure,
5-(2,2-Dibrom-1,1-difluorethoxy)-2-nitro-3,4,6-trifluorbenzoesäure,
5-(1,2-Dichlor-1,2-difluorethoxy)-2-nitro-3,4,6-trifluorbenzoesäure,
5-(2-Chlor-1,1-difluorethoxy)-2-nitro-3,4,6-trifluorbenzoesäure,
5-(2,2,3,3,3-Pentafluorpropoxy)-2-nitro-3,4,6-trifluorbenzoesäure,
5-(2,2,3,3,4,4,4-Heptafluorbutoxy)-2-nitro-3,4,6-trifluorbenzoesäure,
5-Nonafluorbutoxy-2-nitro-3,4,6-trifluorbenzoesäure und
5-Allyloxy-2-nitro-3,4,6-trifluorbenzoesäure,
sowie deren Salze.
Besonders bevorzugt ist 2-Nitro-5-hydroxy-3,4,6-trifluorbenzoesäure und ihre Salze. Unter den Salzen der Verbindung der Formel II werden einerseits die Alkali- und

Erdalkalisalze, also insbesondere die Li-, Na-, K-, Mg- und Ca-Salze, weiterhin die Al-Salze, die Salze mit Uebergangsmetallen wie Fe, Mn, Co, Ni, Cu, und Zn, sowie NH₄^{⊕}-Salze verstanden.

Andererseits handelt es sich um Salze mit organischen Kationen. Zu diesen Kationen gehören insbesondere organische Ammoniumionen wie Triethylammonium, Tributylammonium, Tripropylammonium, Triisopropylammonium, Tetramethylammonium, Tetraethylammonium, Tetrabutylammonium oder Trimethylbenzylammonium; weiterhin können N,N-dialkylierte Aniline wie N,N-Dimethylanilinium als Gegenionen dienen.

Verbindungen der Formel III sind teilweise bekannt (EP-A 271 275, EP-A-352 123). Es handelt sich dabei um solche Verbindungen der Formel III worin R Wasserstoff C₁-C₄-Alkyl oder Halogenmethyl bedeutet. Weitere noch neue und im Zusammenhang mit dem erfindungsgemässen Syntheseverfahren benötigte Ausgangsverbindungen sind beispielsweise:
3-Isoamyloxy-2,4,5-trifluorbenzoesäure,
3-Octyloxy-2,4,5-trifluorbenzoesäure,
3-(1,1,2,2-Tetrafluorethoxy)-2,4,5-trifluorbenzoesäure,
3-(2-Chlor-1,1,2-trifluorethoxy)-2,4,5-trifluorbenzoesäure,
3-(1,1,1,2,3,3,3-Hexafluorpropoxy)-2,4,5-trifluorbenzoesäure,
3-(1,2,3,3,3-Pentafluorpropoxy)-2,4,5-trifluorbenzoesäure,
3-Trifluormethoxy-2,4,5-trifluorbenzoesäure,
3-Chlor-difluormethoxy-2,4,5-trifluorbenzoesäure,
3-(2,2,2-Trifluorethoxy)-2,4,5-trifluorbenzoesäure,
3-(2,2-Dibrom-1,1-difluorethoxy)-2,4,5-trifluorbenzoesäure,
3-(2,2-Dibrom-1,1-difluorethoxy)-2,4,5-trifluorbenzoesäure,
3-(1,2-Dichlor-1,2-difluorethoxy)-2,4,5-trifluorbenzoesäure,
3-(2-Chlor-1,1-difluorethoxy)-2,4,5-trifluorbenzoesäure,
3-(2,2,3,3,3-Pentafluorpropoxy)-2,4,5-trifluorbenzoesäure,
3-Heptafluorpropoxy-2,4,5-trifluorbenzoesäure,
3-(2,2,3,3,4,4,4-Heptafluorbutoxy)-2,4,5-trifluorbenzoesäure,
3-Nonafluorbutoxy-2,4,5-trifluorbenzoesäure oder
3-Allyloxy-2,4,5-trifluorbenzoesäure

Diese Verbindungen können aus 3-Hydroxy-2,4,5-trifluorbenzoesäure durch Veretherung der Hydroxygruppe erhalten werden. Die Reaktionsbedingungen entsprechen denjenigen bei der Herstellung der Verbindungen der Formeln Ib und IIb aus den Verbindungen Ia und IIa.

Ein weiterer Gegenstand der Erfindung ist ein Verfahren zur Herstellung einer Verbindung der Formel (I), dadurch gekennzeichnet, dass eine Verbindung der Formel (III) durch Nitrierung in eine Verbindung der Formel (II) überführt, und diese Verbindung der Formel (II) ohne Aufarbeitung in einem Eintopfverfahren decarboxyliert wird.

Bevorzugt ist ein Verfahren, bei dem die Nitrierung in Wasser bei +10 bis 50 °C mit Salpetersäure, und der anschliessende Decarboxylierungsschritt bei 80 bis 140 °C, gegebenenfalls nach teilweiser Entfernung und/oder Zugabe eines hochsiedenden, mit Wasser mischbaren Lösungsmittels und in Gegenwart einer Base erfolgt.

### Beispiel 1: 4-Hydroxy-2,3,5-trifluornitrobenzol

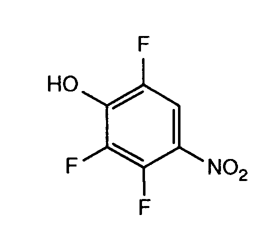

### a1) 2-Nitro-5-hydroxy-3,4,6-trifluorbenzoesäure

Zu einer Suspension von 3 g 3-Hydroxy-2,4,5-trifluorbenzoesäure in 35 ml Chloroform lässt man bei +22°C 1,3 ml Salpetersäure (65 %) zutropfen. Man rührt die Mischung noch 4 Stunden, giesst sie dann auf 200 ml gesättigte Kochsalzlösung und extrahiert dreimal mit je 100 ml Diethylether. Die vereinigten Etherphasen werden dreimal mit je 50 ml gesättigter Kochsalzlösung gewaschen, über Magnesiumsulfat getrocknet und am Rotationsverdampfer eingedampft. Das Rohprodukt wird mit Tetrachlorkohlenstoff verrührt. Man erhält so 3,4 g hellgelbe Kristalle. Durch Rekristallisation aus Toluol wird die reine Titelverbindung mit einem Schmelzpunkt von 158-160°C erhalten.

a2) Zu 30 ml Salpetersäure (65%) werden 57,6 g 3-Hydroxy-2,4,5-trifluorbenzoesäure in 25 ml Wasser gegeben. Man lässt 30 Minuten rühren, giesst die Mischung auf 1000 ml Eiswasser und gibt festes Natriumhydrogencarbonat zu bis der Neutralpunkt erreicht ist. Man stellt mit Salzsäure 37% den pH-Wert auf 1, extrahiert dreimal mit 250 ml Diethylether, vereinigt die Etherphasen, trocknet über Magnesiumsulfat und dampft das Lösungsmittel ab. Durch Rekristallisation aus Toluol wird reine 2-Nitro-5-hydroxy-3,4,6-trifluorbenzoesäure mit einem Schmelzpunkt von 160-162°C erhalten.

b) Zu einer Lösung von 2,65 g 2-Nitro-5-hydroxy-3,4,6-trifluorbenzoesäure in 20 ml Dimethylsulfoxid lässt man 4 ml Triethylamin bei 110°C zutropfen und rührt die Mischung für weitere 2 Stunden. Dann giesst man die Reaktionslösung auf 200 ml Wasser, stellt durch Zusatz von 37%iger Salzsäure den pH-Wert 3 und extrahiert dreimal mit 100 ml Diethylether. Die vereinigten Etherphasen werden dreimal mit 50 ml gesättigter Kochsalzlösung gewaschen, über Magnesiumsulfat getrocknet und am Rotationsverdampfer eingedampft. Man erhält 4-Hydroxy-2,3,5-trifluornitrobenzol mit einem Schmelzpunkt 106-108°C.

## Patentansprüche

1. Verfahren zur Herstellung einer Verbindung der Formel 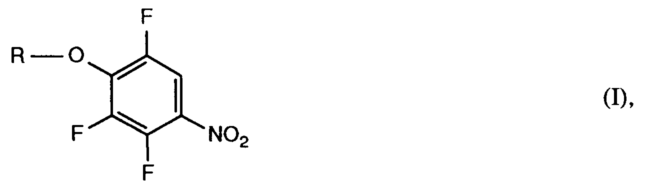 worin R Wasserstoff, C₁-C₈-Alkyl, Halogen-C₁-C₈-alkyl oder Allyl bedeutet, dadurch gekennzeichnet, dass man eine Verbindung der Formel 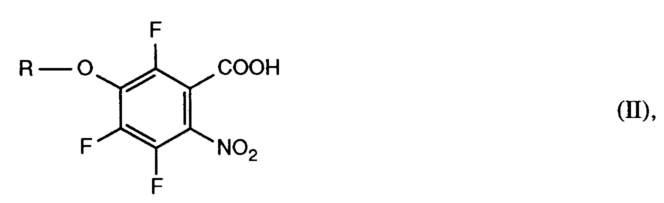 worin R Wasserstoff, C₁-C₈-Alkyl, Halogen-C₁-C₈-alkyl oder Allyl bedeutet, oder eines ihrer Salze, decarboxyliert.

2. Verfahren gemäss Anspruch 1, dadurch gekennzeichnet, dass die Decarboxylierung in Gegenwart einer Base durchgeführt wird.

3. Verfahren gemäss Anspruch 1, dadurch gekennzeichnet, dass die Decarboxylierung in einem organischen Lösungsmittel, einem organischen Lösungsmittel/Wasser-Gemisch oder in Wasser durchgeführt wird.

4. Verfahren gemäss Anspruch 1, dadurch gekennzeichnet, dass die Decarboxylierung bei +50°C bis +250°C durchgeführt wird.

5. Verfahren gemäss Anspruch 1, dadurch gekennzeichnet, dass die Decarboxylierung in Gegenwart einer Base; in einem organischen Lösungsmittel, einem organischen Lösungsmittel/Wasser-Gemisch oder in Wasser; und bei +50°C bis +250°C durchgeführt wird.

6. Verfahren gemäss Anspruch 1, dadurch gekennzeichnet, dass die Decarboxylierung in Dimethylsulfoxid, in Gegenwart von Triethylamin bei +80°C bis +140°C durchgeführt wird.

7. Eine Verbindung der Formel 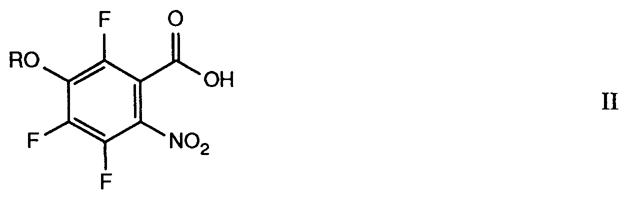 worin R Wasserstoff, C₁-C₈-Alkyl, Halogen-C₂-C₈-alkyl oder Allyl bedeutet, sowie deren Salze.

8. Eine Verbindung der Formel II gemäss Anspruch 7, ausgewählt aus der Gruppe bestehend aus
5-Methoxy-2-nitro-3,4,6-trifluorbenzoesäure,
5-Ethoxy-2-nitro-3,4,6-trifluorbenzoesäure,
5-Propoxy-2-nitro-3,4,6-trifluorbenzoesäure,
5-Isopropoxy-2-nitro-3,4,6-trifluorbenzoesäure,
5-Butoxy-2-nitro-3,4,6-trifluorbenzoesäure,
5-tert.-Butoxy-2-nitro-3,4,6-trifluorbenzoesäure,
5-(1,1,1,2,3,3,3-Hexafluorpropoxy)-2-nitro-3,4,6-trifluorbenzoesäure,
5-(1,1,2,2-Tetrafluorethoxy)-2-nitro-3,4,6-trifluorbenzoesäure,
5-(2-Chlor-1,1,2-trifluorethoxy)-2-nitro-3,4,6-trifluorbenzoesäure,
5-Heptafluorpropoxy-2-nitro-3,4,6-trifluorbenzoesäure,
5-(1,2,3,3,3-Pentafluorpropoxy)-2-nitro-3,4,6-trifluorbenzoesäure,
5-(2,2,2-Trifluorethoxy)-2-nitro-3,4,6-trifluorbenzoesäure,
5-(2,2-Dibrom-1,1-difluorethoxy)-2-nitro-3,4,6-trifluorbenzoesäure,
5-(1,2-Dichlor-1,2-difluorethoxy)-2-nitro-3,4,6-trifluorbenzoesäure,
5-(2-Chlor-1,1-difluorethoxy)-2-nitro-3,4,6-trifluorbenzoesäure,
5-(2,2,3,3,3-Pentafluorpropoxy)-2-nitro-3,4,6-trifluorbenzoesäure,
5-(2,2,3,3,4,4,4-Heptafluorbutoxy)-2-nitro-3,4,6-trifluorbenzoesäure,
5-Nonafluorbutoxy-2-nitro-3,4,6-trifluorbenzoesäure und
5-Allyloxy-2-nitro-3,4,6-trifluorbenzoesäure,
sowie deren Salze.

9. 2-Nitro-5-hydroxy-3,4,6-trifluorbenzoesäure und ihre Salze gemäss Anspruch 7.

10. Verfahren zur Herstellung von einer Verbindung der Formel 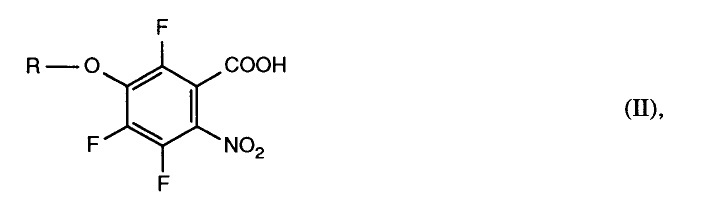 worin R Wasserstoff, C₁-C₈-Alkyl, Halogen-C₁-C₈-alkyl oder Allyl bedeutet, dadurch gekennzeichnet, dass man eine Verbindung der Formel 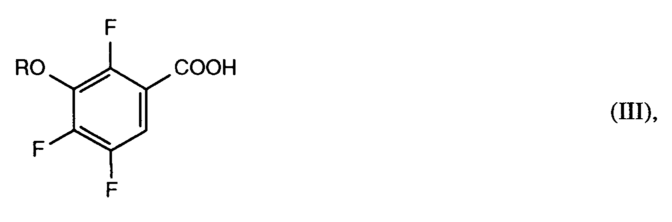 worin R Wasserstoff, C₁-C₈-Alkyl, Halogen-C₂-C₈-alkyl oder Allyl bedeutet, mit einem Nitrierungsmittel umsetzt.

11. Verfahren gemäss Anspruch 10, dadurch gekennzeichnet, dass man als Nitriermittel Nitriersäure verwendet.

12. Verfahren gemäss Anspruch 10, dadurch gekennzeichnet, dass man mit Salpetersäure nitriert.

13. Verfahren gemäss Anspruch 10, dadurch gekennzeichnet, dass man bei einer Temperatur zwischen 0°C bis +60°C nitriert.

14. Verfahren gemäss Anspruch 10, dadurch gekennzeichnet, dass man mit Salpetersäure, in Gegenwart eines Lösungsmittels und bei 0°C bis +60°C nitriert.

15. Verfahren gemäss Anspruch 10, dadurch gekennzeichnet, dass man mit Salpetersäure, in Chloroform und bei +10°C bis +50°C nitriert.

16. Verfahren zur Herstellung einer Verbindung der Formel 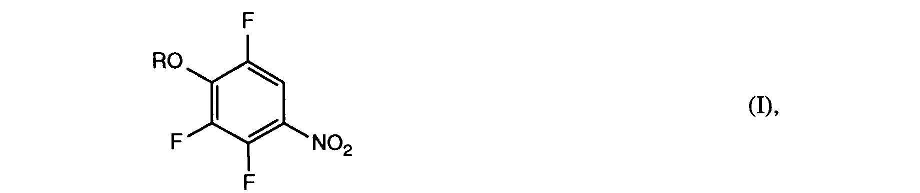 worin R Wasserstoff, C₁-C₈-Alkyl, Halogen-C₁-C₈-alkyl oder Allyl bedeutet, dadurch gekennzeichnet, dass eine Verbindung der Formel 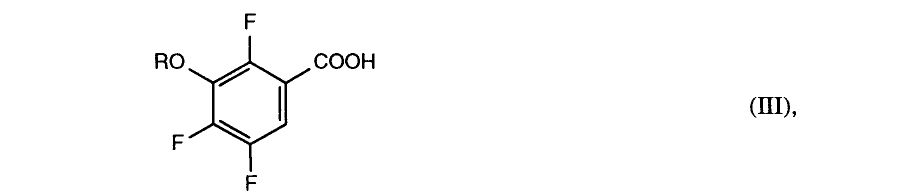 worin R Wasserstoff, C₁-C₈-Alkyl, Halogen-C₁-C₈-alkyl oder Allyl bedeutet, durch Nitrierung in eine Verbindung der Formel 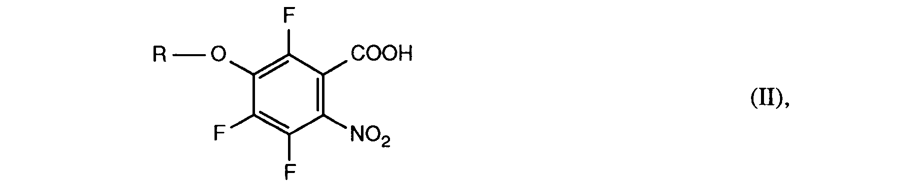 worin R Wasserstoff, C₁-C₈-Alkyl, Halogen-C₁-C₈-alkyl oder Allyl bedeutet, überführt, und diese Verbindung ohne Aufarbeitung in einem Eintopfverfahren decarboxyliert wird.

17. Eine Verbindung der Formel 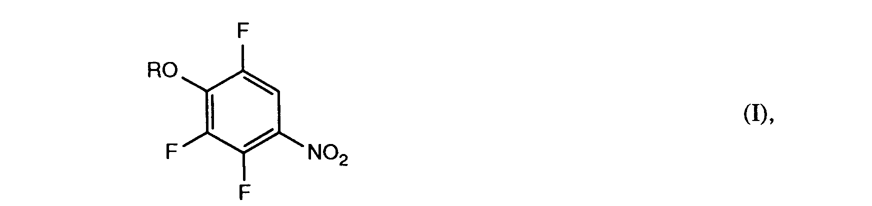 worin R Halogen-C₁-C₈-alkyl oder Allyl bedeutet.

18. Eine Verbindung der Formel I gemäss Anspruch 17, ausgewählt aus der Gruppe bestehend aus
4-(1,1,2,3,3,3-Hexafluorpropoxy)-2,3,5-trifluornitrobenzol,
4-(1,1,2,2-Tetrafluorethoxy)-2,3,5-trifluornitrobenzol,
4-(2-Chlor-1,1,2-trifluorethoxy)-2,3,5-trifluornitrobenzol,
4-(1,2,3,3,3,-Pentafluorpropoxy)-2,3,5-trifluornitrobenzol,
4-Heptafluorpropoxy-2,3,5-trifluornitrobenzol,
4-(2,2,2-Trifluorethoxy)-2,3,5-trifluornitrobenzol,
4-(2,2-Dibrom-1,1-difluorethoxy)-2,3,5-trifluornitrobenzol,
4-(1,2-Dichlor-1,2-difluorethoxy-2,3,5-trifluornitrobenzol,
4-(2-Chlor-1,1-difluorethoxy)-2,3,5-trifluornitrobenzol,
4-(2,2,3,3,3-Pentafluorpropoxy)-2,3,5-trifluornitrobenzol,
4-(2,2,3,3,4,4,4-Heptafluorbutoxy)-2,3,5-trifluornitrobenzol,
4-Nonafluorbutoxy-2,3,5-trifluornitrobenzol,
4-Trifluormethoxy-2,3,5-trifluornitrobenzol und
4-Chlordifluormethoxy-2,3,5-trifluornitrobenzol.
